# EUROPEAN PATENT SPECIFICATION

(11) **EP 0 590 006 B1**
(45) Date of publication and mention of the grant of the patent: **04.09.2002**
(21) Application number: 92912964.1
(22) Date of filing: 01.06.1992
(51) Int. Cl.: C07K 14/785, A61K 38/17

(54) **SYNTHETIC PULMONARY SURFACTANT PEPTIDES**
SYNTHETISCHE PULMONÄRE OBERFLÄCHENAKTIVE PEPTIDE
PEPTIDES TENSIOACTIFS PULMONAIRES SYNTHETIQUES

(30) Priority: 14.06.1991 US 715397
(43) Date of publication of application: 06.04.1994
(73) Proprietor: THE SCRIPPS RESEARCH INSTITUTE, La Jolla, CA 92037 (US)
(72) Inventor: COCHRANE, Charles, G., La Jolla, CA 92037 (US); REVAK, Susan, D., San Diego, CA 92037 (US)
(74) Representative: Fisher, Adrian John
(86) International application number: PCT/US92/04537
(87) International publication number: WO 92/022315

(56) References cited:
- EP-A- 0 348 967
- WO-A-89/06657
- US-A- 4 861 756
- FEBS Letters, Volume 220, Number 2, issued August 1987, ONO et al., "Environment-dependent conformation and antimicrobial activity of gramicidins analog containing leucine and lycine residues", pages 332-336, see especially the Abstract for a disclosure of cyclo (Leu-Lys-Leu-D-Leu-Leu)2.

## Description

### Technical Field

The present invention relates to polypeptides which include a sequence having alternating hydrophobic and hydrophilic amino acid residue regions. In another embodiment, the present invention relates to polypeptides which include a sequence having alternating hydrophobic and positively charged amino acid residue regions. In addition, the present invention relates to polypeptides useful in forming synthetic pulmonary surfactants. The present invention also relates to a recombinant nucleic acid molecule carrying a structural gene that encodes a polypeptide sequence having alternating hydrophobic and hydrophilic (or positively charged) amino acid residue regions. The present invention also discloses the use of such recombinant molecules to produce polypeptides having alternating hydrophobic and hydrophilic (or positively charged) amino acid residue regions.

### Background

Pulmonary surfactant (PS) lines the alveolar epithelium of mature mammalian lungs. Natural PS has been described as a "lipoprotein complex" because it contains both phospholipids and apoproteins that interact to reduce surface tension at the lung air-liquid interface.

Since the discovery of pulmonary surfactant, and the subsequent finding that its deficiency was the primary cause of neonatal respiratory distress syndrome (RDS), a number of studies have been directed towards developing effective surfactant replacement therapy for affected individuals, particularly infants, using exogenous PS. For instance, improvements in lung function as measured by a decrease in mean airway pressure and oxygen requirements have been demonstrated using exogenous surfactants in human pre-term infants. See Hallman, et al, Pediatrics, 71:473-482 (1983); Merritt, et al, J. Pediatr., 108:741-745 (1986); Hallman, et al, J. Pediatr., 106:963-969 (1985); Morley, et al, Lancet, i:64-68 (1981); Merritt, et al, New England J. Med., 315:785-790 (1986), Smyth, et al, Pediatrics, 71:913-917 (1983); Enhorning, et al, Pediatrics, 76:145-153 (1985); Fujiwara, et al, The Lancet, 1:55-59 (1980); Kwong, et al, Pediatrics, 76:585-592 (1985); Shapiro, et al, Pediatrics, 76:593-599 (1985); Fujiwara, in "Pulmonary Surfactant", Robertson, B., Van Golde, L.M.G., Batenburg J. (eds), Elsevier Science Publishers, Amsterdam, pp. 479-503, (1984).

From a pharmacologic point of view, the optimal exogenous PS to use in the treatment of RDS would be one completely synthesized in the laboratory, under controlled and sterile conditions, with negligible batch-to-batch variability in properties. To minimize the possibility of immunologic complications, the apoprotein component of an exogenous PS should be identical to that found in humans. Unfortunately, the composition of naturally occurring PS is complex, and the art has not yet identified all of the biochemical components that generate the biophysical properties needed for high physiologic activity in lungs. In particular, the art has failed to characterize all of the apoproteins present in natural PS or identify the function of the PS apoproteins presently known.

It should be noted that the literature on PS apoproteins and their roles in surfactant function is complex, inconsistent and sometimes contradictory because heterogenous apoprotein preparations were used in many studies. To date, the art has not definitively established the number of different apoproteins present in natural PS.

Of particular interest to the present invention is the use of a polypeptide possessing various characteristics analogous to a low molecular weight (LMW) human PS-associated apoprotein as a component in an exogenous surfactant. Several studies have attempted to isolated or define human PS LMW apoproteins using biochemical techniques. See, for example, Phizackerley, et al, Biochem. J., 183:731-736 (1979), Revak, et al, Am. Rev. Resp. Dis., 134:1258-1265 (1986), Suzuki, et al, Eur. J. Respir. Dis., 69:335-345 (1986), Taeusch, et al, Pediatrics, 77:572-581 (1986), Yu, et al, Biochem. J., 236:85-89 (1986), Whitsett, et al, Pediatric Res., 20:460-467 (1986), Whitsett, et al, Pediatric Res., 20:744-749 (1986), Takahashi, et al, Biochem. Biophys. Res. Comm., 135:527-532 (1986), Suzuki, et al, Exp. Lung. Res., 11:61-73 (1986), Curstedt, et al, Eur. J. Biochem., 168:255-262 (1987), Notter, et al, Chem. Phys. Lipids, 44:1-17 (1987), and Phelps, et al, Am. Rev. Respir. Dis., 135:1112-1117 (1987).

Recently, the art has begun to apply the methods of recombinant DNA technology to overcome the problems associated with not being able to isolate to homogeneity the individual LMW PS apoproteins. For instance, Glasser, et al, Proc. Natl. Acad. Sci., U.S.A., 84:4007-4011 (1987) reported a cDNA derived sequence of amino acid residues that forms at least a portion of a human precursor protein from which at least one mature LMW apoprotein, which they designated SPL (Phe), is formed. While Glasser, et al were not able to determine the carboxy-terminal residue of SPL(Phe), and therefore were not able to identify its complete sequence, they did predict that mature SPL(Phe) was about 60 amino acids in length.

Jacobs, et al, J. Biol. Chem., 262:9808-9811 (1987) have described a cDNA and derived amino acid residue sequence for a human precursor protein similar to that described by Glasser, et al, supra. However, according to Jacobs et al. the mature LMW apoprotein, which they designated PSP-B, formed from the precursor would be 76 amino acid residues in length. In addition, Jacobs, et al, noted that it was not clear that any PS apoprotein derived from the reported precursor protein was present in the surfactant preparations that had been studied clinically by others. The mature apoprotein derived from the precursor protein described by Glasser, et al, supra, and Jacobs, et al, supra, is generally referred to as "SP18", with the monomeric and dimeric forms being referred to as "SP18 monomer" and "SP18 dimer", respectively.

From the foregoing it can be seen that the literature contains multiple nomenclature for what is apparently the same PS apoprotein. In addition, Warr, et al., in Proc. Natl. Acad. Sci., U.S.A. 84: 7915-7919 (1987), described a cDNA derived sequence of 197 amino acid residues that forms a precursor protein from which a mature LMW apoprotein, they designate as SP5, is formed, which seems to indicate that PS contains more than one LMW apoprotein.

Cochrane and Revak, in published PCT patent application no. WO 89/06657, were able to define the mature, biologically active form of SP18 and disclose various SP18-derived polypeptides and analogs which display therapeutically useful surfactant activity.

US4861756 describes a polypeptide-phospholipid complex consisting of alternating hydrophobic/hydrophilic regions which could be used to treat RDS. EP-A-0348967 discloses the synthesis of synthetic pulmonary surfactants consisting of an alpha-helically structured polypeptide linked to a lipid. A method of substituting amino acid residues with leucine or lysine was described by Ono et al., in FEBS Letters 220: 332-336 (1987).

Nevertheless, the biological activity of the above proteins does not match that of the polypeptides disclosed herein, which are useful in therapeutic applications, particularly in forming synthetic pulmonary surfactant compositions.

### SUMMARY OF THE INVENTION

A polypeptide according to the present invention has an amino acid residue sequence represented by the formula
KLLLLKLLLLKLLLLKLLLLK, KLLLLLLLLKLLLLLLLLKLL, or KKLLLLLLLKKLLLLLLLKKL, respectively SEQ ID NOs 7-9.

In another variation, the present invention contempiates a synthetic pulmonary surfactant comprising two pharmaceutically acceptable phospholipids admixed with a fatty acid and a polypeptide having an amino acid residue sequence represented by the formula
KLLLLKLLLLKLLLLKLLLLK, KLLLLLLLLKLLLLLLLLKLL, or KKLLLLLLLKKLLLLLLLKKL, respectively, SEQ ID NOs 7-9. The present invention also contemplates methods of treating respiratory distress syndrome using the disclosed synthetic pulmonary surfactants.

In another embodiment, the present invention contemplates a method of making a therapeutic medicament useful for treating respiratory distress syndrome, comprising admixing one or more pharmaceutically acceptable phospholipids with a therapeutically effective amount of a polypeptide, said polypeptide including a sequence having alternating hydrophobic and hydrophilic amino acid residue regions and having an amino acid residue sequence selected from a group of polypeptides comprising
KLLLLKLLLLKLLLLKLLLLK, KLLLLLLLLKLLLLLLLLKLL, or KKLLLLLLLKKLLLLLLLKKL, respectively SEQ ID NOs 7-9.

In other variations, a polypeptide according to the above formulas, when admixed with said one or more pharmaceutically acceptable phospholipids, forms a synthetic pulmonary surfactant having a surfactant activity greater than the surfactant activity of the phospholipid alone.

Various methods of treating respiratory distress syndrome comprising administering a therapeutically effective amount of a synthetic pulmonary surfactant according to the present invention are also contemplated. In alternative embodiments, a surfactant comprising a polypeptide according to the present invention, admixed with one or more pharmaceutically acceptable phospholipids, is administered. A synthetic surfactant comprising one or more polypeptides according to the within-disclosed formulas may also be therapeutically useful and may be admixed with one or more pharmaceutically acceptable phospholipids prior to administration.

### BRIEF DESCRIPTION OF THE DRAWINGS

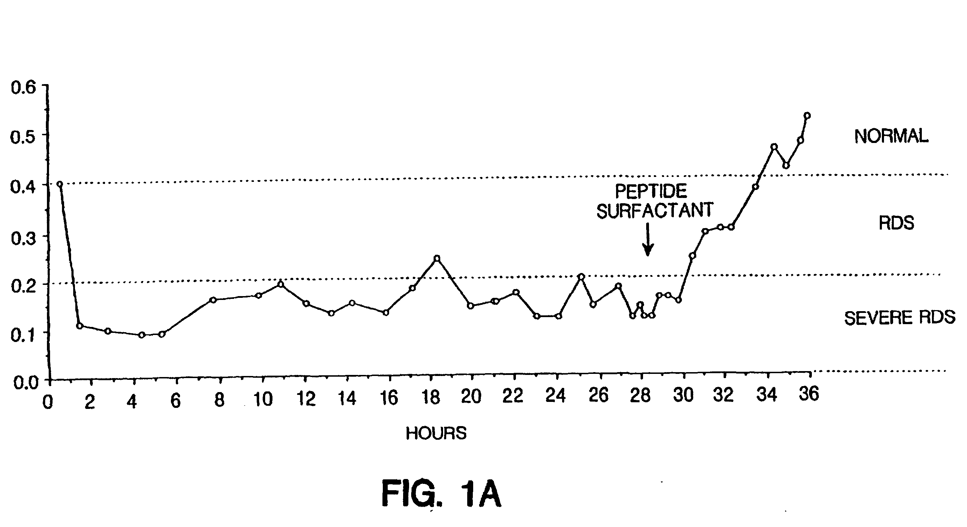

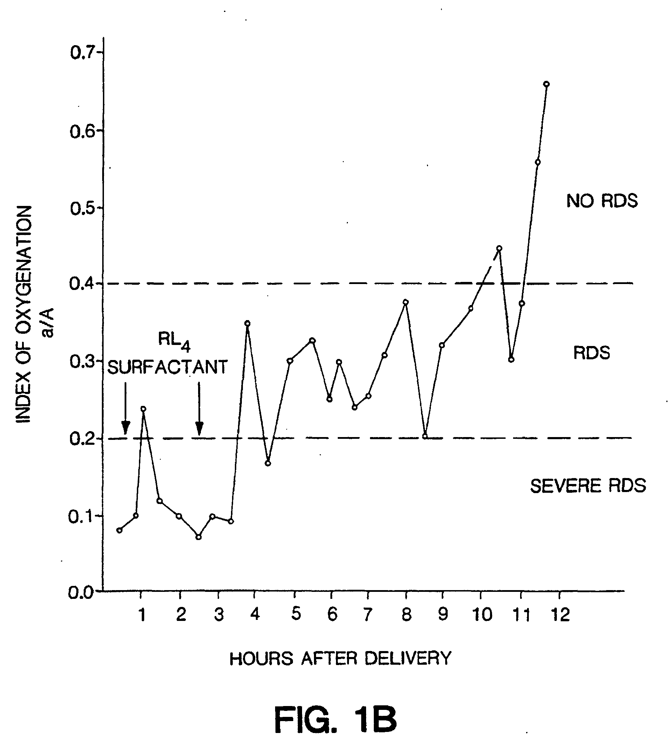

Figure 1 illustrates the effect of administration of RL4-containing surfactant on lung function in premature neonatal monkeys. In Fig. 1A, the index of oxygenation (a/A) is plotted against time subsequent to delivery of the animal, in hours. The surfactant was administered in split dosage, about 28 hours after delivery, as indicated by the arrow. In Fig. 1B, the surfactant was administered in split dosage, during the first 2.5 hours after delivery. The two broken lines separate the graphs into three quadrants, illustrating that the diagnosis of severe respiratory distress syndrome (RDS) applies to a/A values of 0.0 to 0.2; RDS applies to the a/A range 0.2-0.4; and an a/A of 0.4 or better is considered "normal".

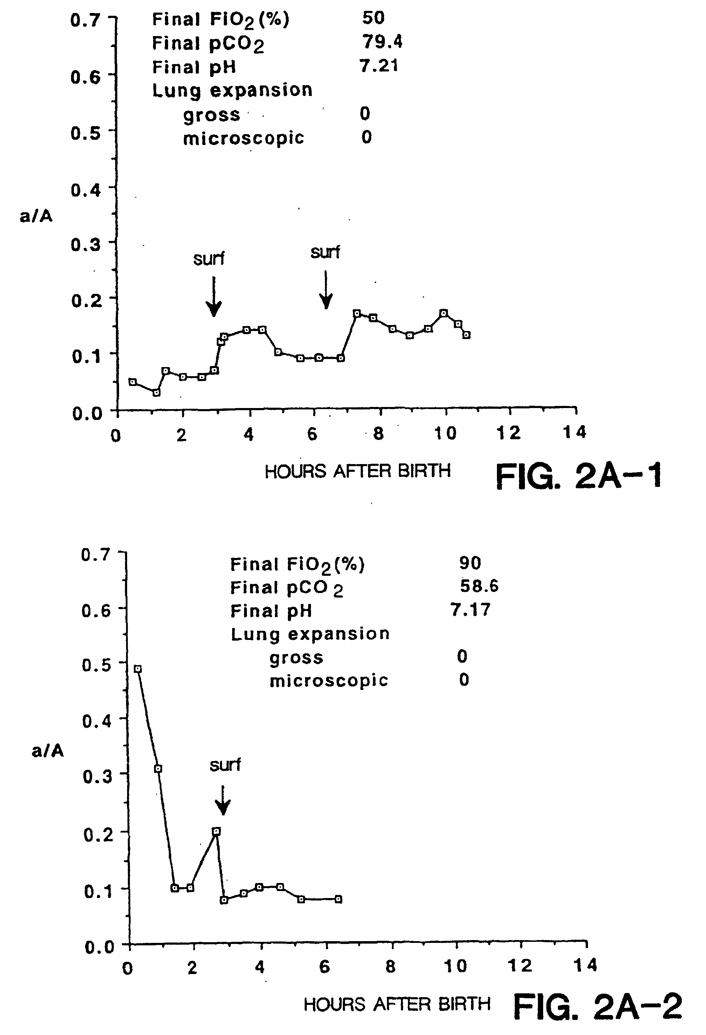

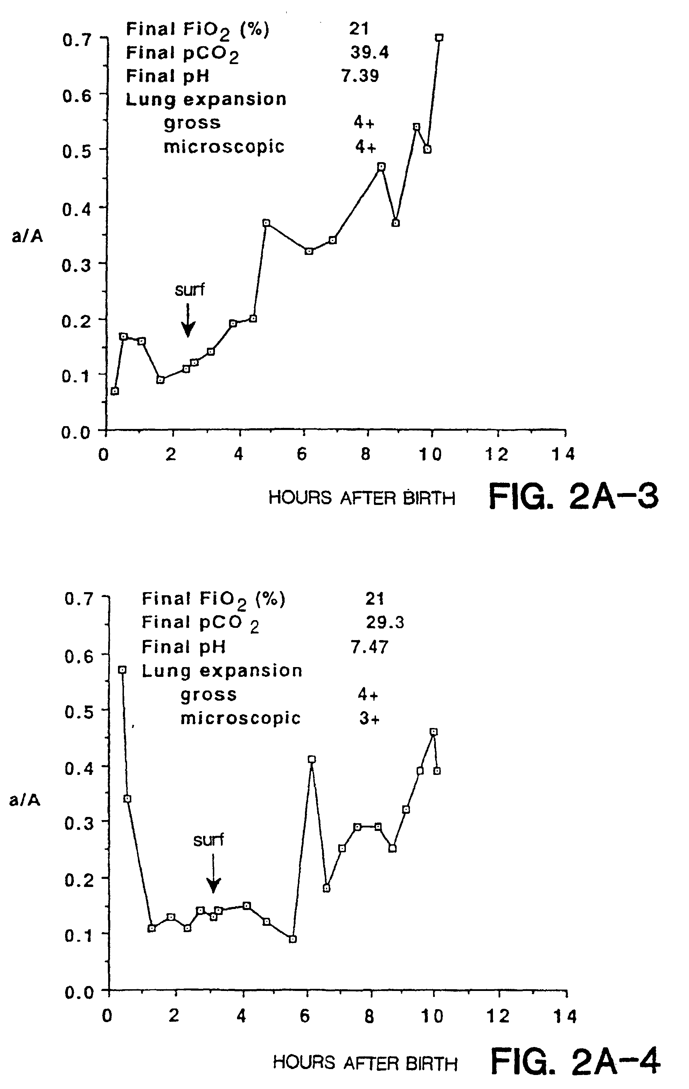

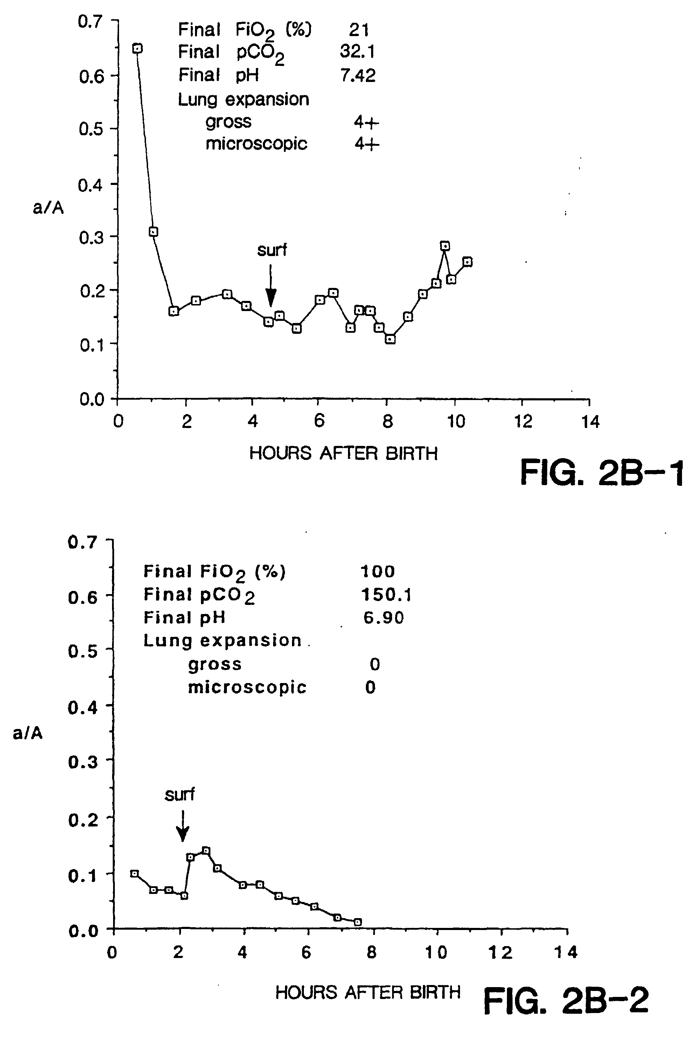

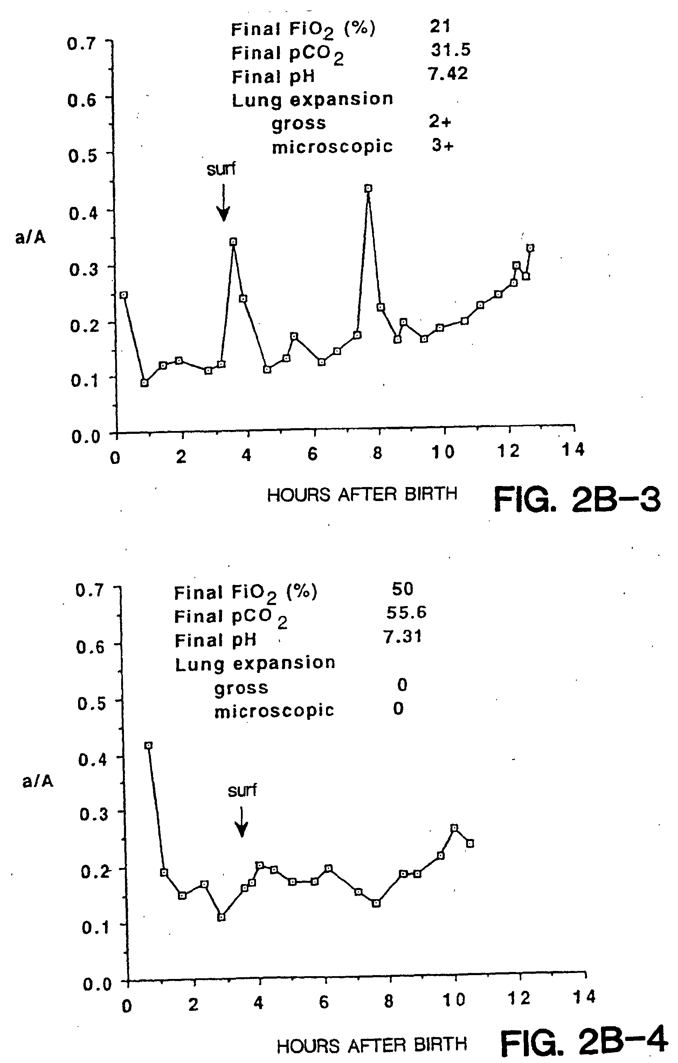

Figure 2 illustrates the effect of KL4-containing synthetic surfactant administration on lung function. In Figs. 2A and 2B, the data for eight monkeys are shown; those which were later confirmed to have received KL4-containing synthetic surfactant were identified as Monkey Nos. 6, 7, 8, and 10 (Figs. 2A-3 & 4, 2B-1 & 3), while those monkeys receiving another surfactant (i.e., one not containing a surfactant peptide of the present invention) were Monkey Nos. 3, 5, 9, and 11 (Figs. 2A-1 & 2, 2B-2 & 4). In all plots, a/A is plotted against hours after birth, with the time of administration of surfactant indicated.

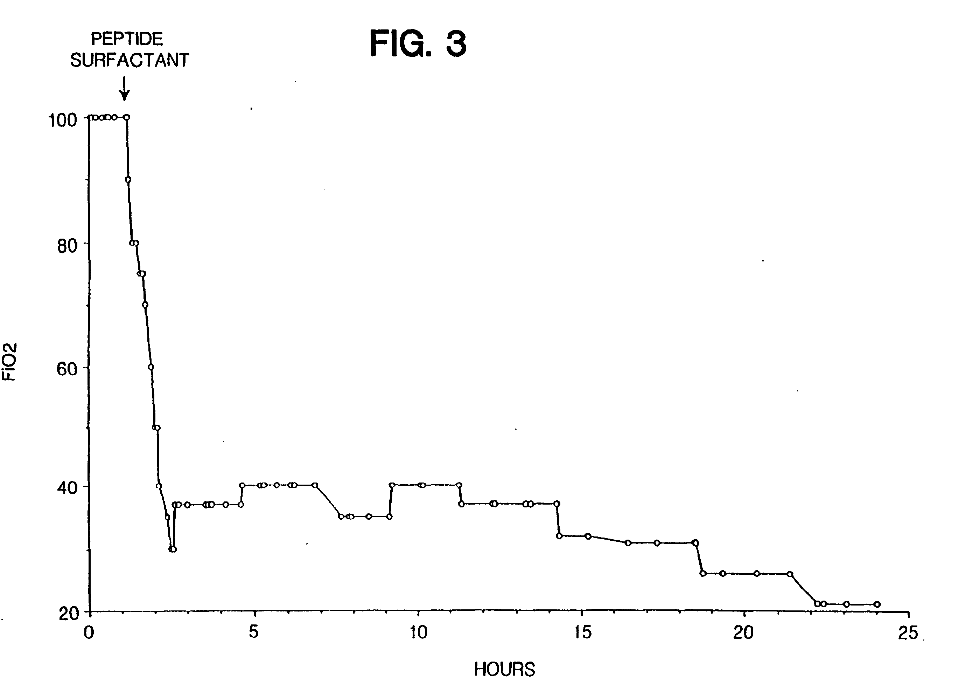

Figure 3 illustrates the gradual withdrawal of oxygen over time (in hours) from a primate delivered and diagnosed with RDS, subsequent to administration of KL4-containing surfactant. FiO₂ is plotted against time in hours. The time at which KL4-containing surfactant was administered is indicated by the arrow. At time zero and 100% inspired oxygen (FiO₂ = 1.0), the animal was receiving 100% oxygen; at 22-25 hours, the animal was receiving 20% oxygen, which is equivalent to room air.

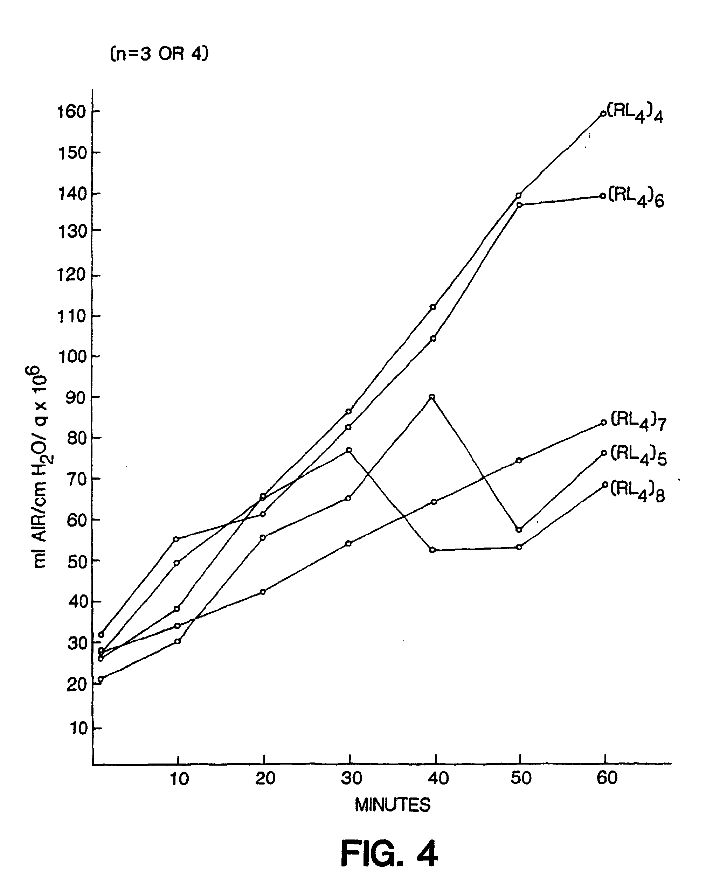

Figure 4 illustrates the results of *in vivo* dynamic compliance tests using synthetic surfactants RL4-CYS, (RL4)₄R, (RL4)₅R, (RL4)₆R, (RL4)₇R, and (RL4)₈R. As noted, n = 3 or 4. The Figure illustrates the effect of administration of various formulations of RL4-containing surfactant on lung function and demonstrates the results of an effort to determine the optimal length of an RL4-containing peptide. The average dynamic compliance value for animals tested using the designated formulation (ml air/cm H₂O/q X 10⁶) is plotted against time in minutes after surfactant administration.

### DETAILED DESCRIPTION OF THE INVENTION

### A. Definitions

Amino Acid: All amino acid residues identified herein are in the natural L-configuration. In keeping with standard polypeptide nomenclature, J. Biol. Chem., 243:3557-59, (1969), abbreviations for amino acid residues are as shown in the following Table of Correspondence:

| TABLE OF CORRESPONDENCE | | |
|---|---|---|
| SYMBOL | | AMINO ACID |
| 1-Letter | 3-Letter | |
| Y | Tyr | L-tyrosine |
| G | Gly | glycine |
| F | Phe | L-phenylalanine |
| M | Met | L-methionine |
| A | Ala | L-alanine |
| S | Ser | L-serine |
| I | Ile | L-isoleucine |
| L | Leu | L-leucine |
| T | Thr | L-threonine |
| V | Val | L-valine |
| P | Pro | L-proline |
| K | Lys | L-lysine |
| H | His | L-histidine |
| Q | Gln | L-glutamine |
| E | Glu | L-glutamic acid |
| W | Trp | L-tryptophan |
| R | Arg | L-arginine |
| D | Asp | L-aspartic acid |
| N | Asn | L-asparagine |
| C | Cys | L-cysteine |

It should be noted that all amino acid residue sequences are represented herein by formulae whose left to right orientation is in the conventional direction of amino-terminus to carboxy-terminus. Furthermore, it should be noted that a dash at the beginning or end of an amino acid residue sequence indicates a bond to a radical such as H and OH (hydrogen and hydroxyl) at the amino- and carboxy-termini, respectively, or a further sequence of one or more amino acid residues. In addition, it should be noted that a virgule (/) at the right-hand end of a residue sequence indicates that the sequence is continued on the next line.

Polypeptide and Peptide: Polypeptide and peptide are terms used interchangeably herein to designate a linear series of no more than about 60 amino acid residues connected one to the other by peptide bonds between the alpha-amino and carboxy groups of adjacent residues.

Protein: Protein is a term used herein to designate a linear series of greater than about 60 amino acid residues connected one to the other as in a polypeptide.

Nucleotide: a monomeric unit of DNA or RNA consisting of a sugar moiety (pentose), a phosphate, and a nitrogenous heterocyclic base. The base is linked to the sugar moiety via the glycosidic carbon (1' carbon of the pentose) and that combination of base and sugar is a nucleoside. When the nucleoside contains a phosphate group bonded to the 3' or 5' position of the pentose it is referred to as a nucleotide.

Base Pair (bp): A partnership of adenine (A) with thymine (T), or of cytosine (C) with guanine (G) in a double stranded DNA molecule.

Conservative substitutions are those where one amino acid residue is replaced by another, biologically similar residue. Examples of conservative substitutions include the substitution of one hydrophobic residue such as isoleucine, valine, leucine or methionine for another, or the substitution of one polar residue for another such as between arginine and lysine, between glutamic and aspartic acids or between glutamine and asparagine and the like. The term "conservative substitution" also includes the use of a substituted amino acid in place of an unsubstituted parent amino acid provided that such a polypeptide also displays the requisite binding activity.

### B. Polypeptides

A polypeptide of the present invention (subject polypeptide) is characterized by its amino acid residue sequence and novel functional properties. A subject polypeptide when admixed with a pharmaceutically acceptable phospholipid forms a synthetic pulmonary surfactant having a surfactant activity greater than the surfactant activity of the phospholipid alone.

In a preferred embodiment, a polypeptide of this invention has amino acid residue sequence that has a composite hydrophobicity of less than zero, preferably less than or equal to -1, more preferably less than or equal to -2. Determination of the composite hydrophobicity value for a peptide is described in detail in Example 1. These hydrophobic polypeptides perform the function of the hydrophobic region of SP18. In one embodiment, the amino acid sequence mimics the pattern of hydrophobic and hydrophilic residues of SP18.

A preferred hydrophobic polypeptide includes a sequence having alternating hydrophobic and hydrophilic amino acid residue regions and is characterized as having at least 10 amino acid residues represented by the formula:

(ZₐU_{b})_{c}Z_{d}

Z and U are amino acid residues such that at each occurrence Z and U are independently selected. Z is a hydrophilic amino acid residue, preferably selected from the group consisting of R, D, E and K. In a preferred embodiment, "Z" is selected from the group.

The polypeptides of the invention are shown in Table 1.

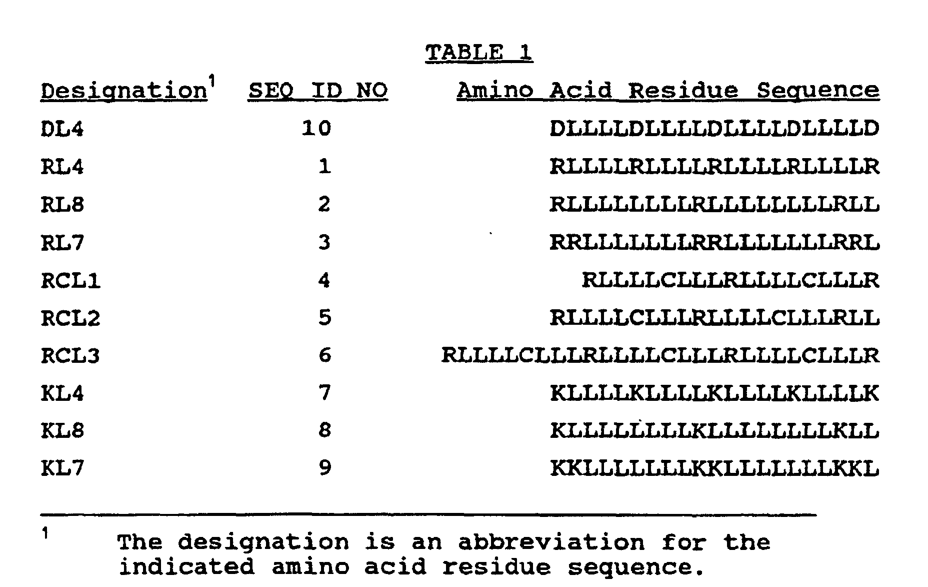

A polypeptide of the present invention can be synthesized by any techniques that are known to those skilled in the polypeptide art. An excellent summary of the many techniques available may be found in J.M. Steward and J.D. Young, "Solid Phase Peptide Synthesis", W.H. Freeman Co., San Francisco, 1969, and J. Meienhofer, "Hormonal Proteins and Peptides", Vol. 2, p. 46, Academic Press (New York), 1983 for solid phase peptide synthesis, and E. Schroder and K. Kubke, "The Peptides", Vol. 1, Academic Press (New York), 1965 for classical solution synthesis.

A subject polypeptide can also be prepared using the solid-phase synthetic technique initially described by Merrifield, in J. Am. Chem. Soc. 85: 2149-2154 (1963). Other polypeptide synthesis techniques may be found, for example, in M. Bodanszky et al., Peptide Synthesis, John Wiley & Sons, 2d Ed., (1976) as well as in other reference works known to those skilled in the art. A summary of polypeptide synthesis techniques may be found in J. Stuart and J.D. Young, Solid Phase Peptide Synthesis, Pierce Chemical Company, Rockford, IL, 3d Ed., Neurath, H. et al., Eds., p. 104-237, Academic Press, New York, NY (1976). Appropriate protective groups for use in such syntheses will be found in the above texts as well as in J.F.W. McOmie, Protective Groups in Organic Chemistry, Plenum Press, New York, NY (1973).

In general, these methods comprise the sequential addition of one or more amino acid residues or suitably protected amino acid residues to a growing peptide chain. Normally, either the amino or carboxyl group of the first amino acid residue is protected by a suitable, selectively removable protecting group. A different, selectively removable protecting group is utilized for amino acids containing a reactive side group such as lysine.

Using a solid phase synthesis as exemplary, the protected or derivatized amino acid is attached to an inert solid support through its unprotected carboxyl or amino group. The protecting group of the amino or carboxyl group is then selectively removed and the next amino acid in the sequence having the complimentary (amino or carboxyl) group suitably protected is admixed and reacted under conditions suitable for forming the amide linkage with the residue already attached to the solid support. The protecting group of the amino or carboxyl group is then removed from this newly added amino acid residue, and the next amino acid (suitably protected) is then added, and so forth. After all the desired amino acids have been linked in the proper sequence, any remaining terminal and side group protecting groups (and solid support) are removed sequentially or concurrently, to afford the final polypeptide.

A polypeptide of the present invention may be a composite polypeptide, further including additional amino acid residues at the amino- or carboxy-terminal end. Said additional sequences may serve to enhance expression of the polypeptide or may serve as a "linker" sequence, but preferably do not decrease or otherwise interfere with the biological activity of a polypeptide of the present invention.

Amino acid residue linkers are usually at least one residue and can be 40 or more residues, more often 1 to 10 residues. Typical amino acid residues used for linking are tyrosine, cysteine, lysine, glutamic and aspartic acid, or the like. In addition, a polypeptide sequence of this invention can differ from the natural sequence by the sequence being modified by terminal-NH₂ acylation, e.g., acetylation, or thioglycolic acid amidation, terminal-carboxlyamidation, e.g., ammonia, methylamine, etc.

When coupled to a carrier via a linker to form what is known in the art as a carrier-hapten conjugate, a polypeptide of the present invention is capable of inducing antibodies that immunoreact with synthetic surfactants of the present invention. In view of the well established principle of immunologic cross-reactivity, the present invention therefore contemplates antigenically related variants of the polypeptides shown in Table 1. An "antigenically related variant" is a polypeptide that includes at least a six amino acid residue sequence portion of a polypeptide from Table 1 and which is capable of inducing antibody molecules that immunoreact with a polypeptide from Table 1 and a synthetic surfactant containing same.

### C. Nucleic Acid Segments

In living organisms, the amino acid residue sequence of a protein or polypeptide is directly related via the genetic code to the deoxyribonucleic acid (DNA) 'sequence of the structural gene that codes for the protein. Thus, a structural gene can be defined in terms of the amino acid residue sequence, i.e., protein or polypeptide, for which it codes.

An important and well known feature of the genetic code is its redundancy. That is, for most of the amino acids used to make proteins, more than one coding nucleotide triplet (codon) can code for or designate a particular amino acid residue. Therefore, a number of different nucleotide sequences may code for a particular amino acid residue sequence. Such nucleotide sequences are considered functionally equivalent since they can result in the production of the same amino acid residue sequence in all organisms. Occasionally, a methylated variant of a purine or pyrimidine may be incorporated into a given nucleotide sequence. However, such methylations do not affect the coding relationship in any way.

A DNA segment of the present invention is characterized as consisting essentially of a DNA sequence that encodes a polypeptide according to the within-disclosed formulas. That is, a DNA segment of the present invention forms a structural gene capable of expressing a polypeptide with alternating hydrophobic and hydrophilic amino acid residue regions. While the codons of the DNA segment need not be collinear with the amino acid residue sequence of a presently-disclosed polypeptide because of the presence of an intron, it is preferred that the structural gene be capable of expressing said polypeptides in mature form, i.e., without the need for post-translational proteolytic processing. Preferably, the gene is present as an uninterrupted linear series of codons where each codon codes for an amino acid residue found in a polypeptide of the present invention, i.e., a gene containing no introns.

### D. Recombinant Nucleic Acid Molecules

A subject polypeptide can also be prepared using recombinant nucleic acid methodologies well known in the art. For instance, DNA sequences useful in producing a subject polypeptide are described in Paik et al., PNAS USA 82: 3445-3449, (1985); McLean et al., J. Biol. Chem. 259: 6498-6504, (1984); and Rall et al., J. Biol. Chem. 257: 4171-4178, (1982). A DNA segment coding for a polypeptide of this invention can be synthesized by chemical techniques, for example the phosphotriester method of Matteucci et al., J. Am. Chem. Soc. 103: 3185, (1981). The DNA segment can then be ligated into an expression vector, and a host transformed therewith can be used to produce the polypeptide. See, for example, Current Protocols In Molecular Biology, Ausubel et al., eds., John Wiley & Sons, New York, NY; and U.S. Patents No. 4,237,224 and No. 4,356,270, the disclosures of which are hereby incorporated by reference. Of course, by chemically synthesizing the coding sequence, any desired modifications can be made simply by substituting the appropriate bases for those encoding the native amino acid residue sequence.

The recombinant expression vectors capable of expressing a subject polypeptide and methods of their use for producing a subject polypeptide are contemplated as part of the present invention.

Also contemplated by the present invention are ribonucleic acid (RNA) equivalents of the above described DNA segments.

Nucleic acid molecules may also be synthesized via amplification methods, also known as the polymerase chain reaction ("PCR") methodology. PCR amplification methods are described in detail in U.S. Patent Nos. 4,683,192, 4,683,202, 4,800,159, and 4,965,188, and at least in several texts including "PCR Technology: Principles and Applications for DNA Amplification", H. Erlich, ed., Stockton Press, New York (1989); and "PCR Protocols: A Guide to Methods and Applications", Innis et al., eds., Academic Press, San Diego, California (1990), the disclosures of which are incorporated herein by reference. Other methods and primers are described in Nilsson, et al., Cell 58: 707 (1989), Ennis, et al., PNAS USA 87: 2833-7 (1990), and Zemmour, et al., Immunogenetics 33: 310-20 (1991), for example. Restriction sites may also be incorporated into the 5' and 3' primers to enable the amplification products to be subcloned into sequencing or expression vectors. It may also be helpful to place a 4-base spacer sequence proximal to the restriction site to improve the efficiency of cutting amplification products with enzymes.

The recombinant nucleic acid molecules of the present invention can be produced by operatively linking a vector to a nucleic acid segment of the present invention.

As used herein, the phase "operatively linked" means that the subject nucleic acid segment is attached to the vector so that expression of the structural gene formed by the segment is under the control of the vector.

As used herein, the term "vector" refers to a nucleic acid molecule capable of replication in a cell and to which another nucleic acid segment can be operatively linked so as to bring about replication of the attached segment. Vectors capable of directing the expression of a structural gene coding for a polypeptide of the present invention are referred to herein as "expression vectors." Thus, a recombinant nucleic acid molecule (rDNA or rRNA) is a hybrid molecule comprising at least two nucleotide sequences not normally found together in nature.

The choice of vector to which a nucleic acid segment of the present invention is operatively linked depends directly, as is well known in the art, on the functional properties desired, e.g., protein expression, and the host cell to be transformed, these being limitations inherent in the art of constructing recombinant nucleic acid molecules. However, a vector contemplated by the present invention is at least capable of directing the replication, and preferably also expression, of a structural gene encoding a polypeptide of the present invention included in a nucleic acid segment to which it is operatively linked.

In preferred embodiments, a vector contemplated by the present invention includes a procaryotic replicon, i.e., a DNA sequence having the ability to direct autonomous replication and maintenance of an rDNA molecule extrachromosomally in a procaryotic host cell, such as a bacterial host cell, transformed therewith. Such replicons are well known in the art. In addition, those embodiments that include a procaryotic replicon also include a gene whose expression confers drug resistance to a bacterial host transformed therewith. Typical bacterial drug resistance genes are those that confer resistance to ampicillin or tetracycline.

Those vectors that include a procaryotic replicon can also include a procaryotic promoter capable of directing the expression (transcription and translation) of a gene in a bacterial host cell, such as *E. coli*, transformed therewith. A promoter is an expression control element formed by a DNA sequence that permits binding of RNA polymerase and transcription to occur. Promoter sequences compatible with bacterial hosts are typically provided in plasmid vectors containing convenient restriction sites for insertion of a DNA segment. Typical of such vector plasmids are pUC8, pUC9, pBR322 and pBR329 available from Biorad Laboratories, (Richmond, CA) and pPL and pKK223 available from Pharmacia, Piscataway, N.J.

Expression vectors compatible with eucaryotic cells, preferably those compatible with vertebrate cells, can also be used to form an rDNA molecule of the present invention. Eucaryotic cell expression vectors are well known in the art and are available from several commercial sources. Typically, such vectors are provided containing convenient restriction sites for insertion of the desired DNA segment. Typical of such vectors are pSVL and pKSV-10 (Pharmacia), pBPV-1/pML2d (International Biotechnologies, Inc.), and pTDT1 (ATCC, #31255).

In preferred embodiments, a aucaryotic cell expression vector used to construct an rDNA molecule of the present invention includes a selection marker that is effective in a eucaryotic cell, preferably a drug resistance selection marker. A preferred drug resistance marker is the gene whose expression results in neomycin resistance, i.e., the neomycin phosphotransferase (neo) gene. Southern, et al, J. Mol. Appl. Genet., 1:327-341 (1982).

The use of retroviral expression vectors to form a recombinant nucleic acid molecule of the present invention is also contemplated. As used herein, the term "retroviral expression vector" refers to a nucleic acid molecule that includes a promoter sequence derived from the long terminal repeat (LTR) region of a retrovirus genome.

In preferred embodiments, the expression vector is a retroviral expression vector that is replication-incompetent in eucaryotic cells. The construction and use of retroviral vectors has been described by Sorge, et al, Mol. Cell. Biol., 4:1730-37 (1984).

A variety of methods have been developed to operatively link nucleic acid segments to vectors via complementary cohesive termini. For instance, complementary homopolymer tracts can be added to the nucleic acid segment to be inserted and to a terminal portion of the vector nucleic acid. The vector and nucleic acid segment are then joined by hydrogen bonding between the complementary homopolymeric tails to form a recombinant nucleic acid molecule.

Synthetic linkers containing one or more restriction sites provide an alternative method of joining a nucleic acid segment to vectors. For instance, a DNA segment of the present invention is treated with bacteriophage T4 DNA polymerase or *E. coli* DNA polymerase I, enzymes that remove protruding, 3', single-stranded termini with their 3'-5' exonucleolytic activities and fill in recessed 3' ends with their polymerizing activities. The combination of these activities therefore generates blunt-ended DNA segments. The blunt-ended segments are then incubated with a large molar excess of linker molecules in the presence of an enzyme that is able to catalyze the ligation of blunt-ended DNA molecules, such as bacteriophage T4 DNA ligase. Thus, the products of the reaction are DNA segments carrying polymeric linker sequences at their ends. These DNA segments are then cleaved with the appropriate restriction enzyme and ligated to an expression vector that has been cleaved with an enzyme that produces termini compatible with those of the DNA segment.

Synthetic linkers containing a variety of restriction endonuclease sites are commercially available from a number of sources including International Biotechnologies, Inc., New Haven, CT.

Also contemplated by the present invention are RNA equivalents of the above described recombinant DNA molecules.

### E. Transformed Cells and Cultures

The present invention also relates to a host cell transformed with a recombinant nucleic acid molecule of the present invention, preferably an rDNA capable of expressing a polypeptide according to the present invention. The host cell can be either procaryotic or eucaryotic.

"Cells" or "transformed host cells" or "host cells" are often used interchangeably as will be clear from the context. These terms include the immediate subject cell, and, of course, the progeny thereof. It is understood that not all progeny are exactly identical to the parental cell, due to chance mutations or differences in environment. However, such altered progeny are included when the above terms are used.

Bacterial cells are preferred procaryotic host cells and typically are a strain of *E. coli* such as, for example, the *E. coil* strain DH5 available from Bethesda Research Laboratories, Inc., Bethesda, MD. Preferred eucaryotic host cells include yeast and mammalian cells, preferably vertebrate cells such as those from a mouse, rat, monkey or human fibroblastic cell line. Preferred eucaryotic host cells include Chinese hamster ovary (CHO) cells available from the ATCC as CCL61 and NIH Swiss mouse embryo cells NIH/3T3 available from the ATCC as CRL 1658. Transformation of appropriate cell hosts with a recombinant nucleic acid molecule of the present invention is accomplished by well known methods that typically depend on the type of vector used. With regard to transformation of procaryotic host cells, see, for example, Cohen, et al, Proc. Natl. Acad. Sci. USA, 69:2110 (1972); and Maniatis, et al, Molecular Cloning, A Laboratory Manual, Cold Spring Harbor Laboratory, Cold Spring Harbor, NY (1982). With regard to transformation of vertebrate cells with recombinant nucleic acid molecules containing retroviral vectors, see, for example, Sorge, et al, Mol. Cell. Biol., 4:1730-37 (1984); Graham, et al, Virol., 52:456 (1973); and Wigler, et al, Proc. Natl. Acad. Sci. USA, 76:1373-76 (1979).

Successfully transformed cells, i.e., cells that contain a recombinant nucleic acid molecule of the present invention, can be identified by well known techniques. For example, cells resulting from the introduction of an rDNA of the present invention can be cloned to produce monoclonal colonies. Cells from those colonies can be harvested, lysed and their DNA content examined for the presence of the rDNA using a method such as that described by Southern, J. Mol. Biol., 98:503 (1975) or Berent, et al, Biotech., 3:208 (1985).

In addition to directly assaying for the presence of rDNA, successful transformation can be confirmed by well known immunological methods when the rDNA is capable of directing the expression of a polypeptide according to the present invention. For example, cells successfully transformed with an expression vector operatively linked to a DNA segment of the present invention produce polypeptides displaying antigenicity. Thus, a sample of a cell culture suspected of containing transformed cells are harvested and assayed for a polypeptide having alternating hydrophobic and hydrophilic amino acid residue regions according to the present invention, using antibodies specific for that antigen, the production and use of such antibodies being well known in the art.

Thus, in addition to the transformed host cells themselves, the present invention also contemplates a culture of those cells, preferably a monoclonal (clonally homogeneous) culture, or a culture derived from a monoclonal culture, in a nutrient medium. Preferably, the culture also contains a polypeptide or protein displaying the aforementioned antigenicity, and more preferably, a biologically active polypeptide useful in surfactant compositions of the present invention.

Nutrient media useful for culturing transformed host cells are well known in the art and can be obtained from several commercial sources. In embodiments wherein the host cell is mammalian, a "serum-free" medium is preferably used.

### F. Recombinant Methods for Producing Polypeptides

Another aspect of the present invention pertains to a method for producing polypeptides, preferably polypeptides displaying surfactant activity. The method entails initiating a culture comprising a nutrient medium containing host cells, preferably human cells, transformed with a rDNA molecule of the present invention that is capable of expressing a polypeptide according to the present invention. The culture is maintained for a time period sufficient for the transformed cells to express said polypeptide. The expressed protein or polypeptide is then recovered from the culture.

Methods for recovering an expressed protein from a culture are well known in the art and include fractionation of the protein-containing portion of the culture using well known biochemical techniques. For instance, the methods of gel filtration, gel chromatography, ultrafiltration, electrophoresis, ion exchange, affinity chromatography and the like, such as are known for protein fractionations, can be used to isolate the expressed proteins found in the culture. In addition, immunochemical methods, such as immunoaffinity, immunoadsorption and the like can be performed using well known methods.

Also contemplated by the present invention is a polypeptide, preferably a polypeptide displaying surfactant activity, produced by a recombinant nucleic acid method described herein.

### G. Polypeptide-Containing Compositions

The present invention contemplates a polypeptide-containing composition (subject polypeptide or protein composition) wherein the polypeptide is present in either substantially isolated or substantially pure form. By "isolated" is meant that the polypeptide is present as part of a composition free of other polypeptides or proteins displaying surfactant activity.

By "substantially pure" is meant that a subject polypeptide is present as part of a composition free of other polypeptides or proteins.

"Surfactant activity" for a protein or polypeptide is defined as the ability, when combined with lipids, either alone or in combination with other proteins, to exhibit activity in the *in vivo* assay of Robertson, Lung, 158:57-68 (1980). In this assay, the sample to be assessed is administered through an endotracheal tube to fetal rabbits or lambs delivered prematurely by Caesarian section. (These "preemies" lack their own PS, and are supported on a ventilator.) Measurements of lung compliance, blood gases and ventilator pressure provide indices of activity. Preliminary assessment of activity may also be made by an in vitro assay, for example that of King, et al, Am. J. Physiol., 223:715-726 (1972), or that illustrated below which utilizes a measurement of surface tension at a air-water interface when a protein or polypeptide is admixed with a phospholipid.

### H. Synthetic Surfactants

Recombinantly produced polypeptides displaying surfactant activity and/or a subject polypeptide can be admixed with a pharmaceutically acceptable phospholipid to form a synthetic pulmonary surfactant (PS) useful in the treatment of respiratory distress syndrome.

The phase "pharmaceutically acceptable" refers to molecular entities and compositions that do not produce an allergic or similar untoward reaction when administered to a human.

Phospholipids useful in forming synthetic alveolar surfactants by admixture with protein are well known in the art. See, Notter, et al, Clin. Perinatology, 14:433-79 (1987), for a review of the use of both native and synthetic phospholipids for synthetic surfactants.

In one embodiment, the present invention contemplates a synthetic pulmonary surfactant effective in treating RDS comprising an effective amount of a subject polypeptide admixed with a pharmaceutically acceptable phospholipid. While methods for determining the optimal polypeptidé:phospholipid weight ratios for a given polypeptide-phospholipid combination are well known, therapeutically effective ratios are in the range of about 1:5 to about 1:10,000, preferably about 1:100 to about 1:5,000, and more preferably about 1:500 to about 1:1000. In a more preferred embodiment, the polypeptide:phospholipid weight ratio is in the range of about 1:5 to about 1:2,000, preferably about 1:7 to about 1:1,000, and more preferably about 1:10 to about 1:100. Thus, a synthetic pulmonary surfactant of this invention can contain about 50, usually about 80, to almost 100 weight percent lipid and about 50, usually about 20, to less than 1 weight percent polypeptide. Preferably a subject polypeptide is about 1 to about 10 weight percent of the surfactant for polypeptides having alternating hydrophobic and hydrophilic amino acid residue regions.

The lipid portion is preferably about 50 to about 90, more preferably about 50 to about 75, weight percent dipalmitoylphosphatidylcholine (DPPC) with the remainder unsaturated phosphatidyl choline, phosphatidyl glycerol (PG), triacylglycerols, palmitic acid sphingomyelin or admixtures thereof. The synthetic pulmonary surfactant is prepared by admixing a solution of a subject polypeptide with a suspension of liposomes or by admixing the subject polypeptide and lipids directly in the presence of organic solvent. The solvent is then removed by dialysis or evaporation under nitrogen and/or exposure to vacuum.

A subject synthetic pulmonary surfactant is preferably formulated for endotracheal administration, e.g., typically as a liquid suspension, as a dry powder "dust", or as an aerosol. For instance, a synthetic surfactant (polypeptide-lipid micelle) is suspended in a liquid with a pharmaceutically acceptable excipient such as water, saline, dextrose, glycerol and the like. A surfactant-containing therapeutic composition can also contain small amounts of non-toxic auxiliary substances such as pH buffering agents including sodium acetate, sodium phosphate and the like. To prepare a synthetic surfactant in dust form, a synthetic surfactant is prepared as described herein, then lyophilized and recovered as a dry powder.

If it is to be used in aerosol administration, a subject synthetic surfactant is supplied in finely divided form along with an additional surfactant and propellent. Typical surfactants which may be administered are fatty acids and esters. However, it is preferred, in the present case, to utilize the other components of the surfactant complex DPPC and PG. Useful propellants are typically gases at ambient conditions, and are condensed under pressure. Lower alkane and fluorinated alkane, such as Freon, may be used. The aerosol is packaged in a container equipped with a suitable valve so that the ingredients may be maintained under pressure until released.

A synthetic surfactant is administered, as appropriate to the dosage form, by endotracheal tube, by aerosol administration, or by nebulization of the suspension or dust into the inspired gas. Amounts of synthetic PS between about 0.1 mg and about 100 mg, preferably about 70 mg to about 90 mg, are administered in one dose. For use in newly born infants, one or two administrations are generally sufficient. For adults, sufficient reconstituted complex is administered to produce a PO₂ within the normal range (Hallman, et al, J. Clinical Investigation, 70:673-682, 1982).

The following examples are intended to illustrate, but not limit, the present invention. Some examples show compounds that are illustrative of, but not included in the present invention.

### EXAMPLE 1

### In Vitro Assessment of Polypeptide Surfactant Activity

### A. Methods

### 1. Measurement of Surfactant Activity

Measurements of surface pressure across an air-liquid interface (expressed in negative cm of H₂O pressure) at minimal (8 min) bubble radius were determined at various times using the pulsating bubble technique described by Enhorning, J. Appl. Physiol., 43:198-203 (1977).

Briefly, the Enhorning Surfactometer (Surfactometer International, Toronto, Ontario) measures the pressure gradient (ΔP) across a liquid-air interface of a bubble that pulsates at a rate of 20 cycles/min between a maximal (0.55 mm) and minimal (0.4 mm) radius. The bubble, formed in a 37°C, water-enclosed, 20-µl sample chamber, is monitored through a microscopic optic while the pressure changes are recorded on a strip chart recorder calibrated for 0 and -2 cm H₂O.

### 2. Determination of Composite Hydrophobicity Value

The composite hydrophobicity value of each peptide was determined by assigning to each amino acid residue in a peptide its corresponding hydrophilicity value as described in Table 1 of Hopp, et al, Proc. Natl. Acad. Sci., U.S.A., 78:3824-3829 (1981), which disclosure is incorporated herein by reference. For a given peptide, the hydrophilicity values were summed, the sum representing the composite hydrophobicity value.

### 3. Preparation of Synthetic Surfactants

After admixture with solvent, each peptide was combined with phospholipids (DPPC:PG), 3:1 to form a synthetic surfactant according to one of the following methods.

Method A was accomplished by admixing 16 µl of peptide/solvent admixture (40 µg peptide) with 100 µl of chloroform containing 400 µg phospholipids, agitating the admixture for about 10 at 37°C to form a peptide/phospholipid admixture. Chloroform was removed from the peptide/phospholipid admixture by drying under N₂. The synthetic surfactant thus formed was then admixed with 90 µl of H₂O and gently agitated for about 10 minutes at 37°C. Subsequently, 10 µl of 9% NaCl was admixed to the surfactant containing solution.

Method B was accomplished by first placing 100 µl of chloroform containing 400 µg of phospholipids in a glass tube and removing the chloroform by drying under N₂ for about 10 minutes at 37°C. Sixteen µl of peptide/solvent admixture and 74 µl H₂O were admixed with the dried phospholipids, and then gently agitated for about 10 minutes at 37°C. To the synthetic surfactant thus formed was admixed 10 µl of 9% NaCl.

Method C was accomplished by first maintaining the polypeptide-PL admixture at 43°C for 10 minutes, after which time the solvents were removed from the admixture by drying under N₂. When needed, admixtures were further dried by 15 minutes exposure to vacuum to form a dried polypeptide-PL admixture. Water was then admixed with each dried admixture in an amount calculated to equal 90% of the volume necessary to give a final PL concentration of either 5 or 10 mg/ml to form a second admixture. This second admixture was maintained for one hour at 43°C with agitation. Subsequently, a volume of 6% NaCl equal to 10% of the volume necessary to give the desired PL concentration was admixed with the second admixture and the resulting final admixture was maintained for 10 minutes at 43°C. In most cases, the final admixture was subjected to a last step of 3 cycles of freezing and thawing.

### 4. Octylglucopyranoside Assay

An assay for the quantitation of n-octyl-beta-D-glucopyranoside, based on the anthrone method of Spiro, Methods Enzymol., 8:3-5 (1966) has been described previously by Revak, et al, Am. Rev. Respir. Dis., 134:1258-1265 (1986).

### 5. Protein Determinations

Organic samples containing up to 5 µg protein were dried in 12x75 mm glass tubes under nitrogen. Fifteen microliters (µl) of 1% SDS in H₂O and 300 µl BCA Protein Assay Reagent (Pierce Chemical Co., Rockford, IL) were admixed with the protein in each tube. Tubes were covered and incubated at 60°C for 30 min. After cooling, the samples were transferred to a 96-well flat-bottom polystyrene microtiter plate and OD₅₅₀ measured. Bovine serum albumin was used as a standard. It should be noted that some phospholipids will react in the BCA protein assay making protein quantitations inaccurate when lipid is present (i.e., prior to Bio-Sil HA chromatography). Additionally, once purified, the hydrophobic LMW apoproteins themselves react poorly with the BCA reagents and all quantitations of the isolated proteins were, therefore, based on amino acid compositions.

### 6. Phospholipids

Dipalmitoylphosphatidylcholine (DPPC, beta, gamma-dipalmitoyl-L-alpha-lecithin) and L-alpha-phosphatidyl-DL-glycerol (PG, derivative of egg lecithin) were purchased from either Calbiochem-Behring (La Jolla, CA) or Avanti Polar-Lipids, Inc. (Birmingham, AL). DPPC was added to PG in chloroform in a weight ratio of 3:1.

### 7. Surfactant Activity Assays

*In vitro* assays of surfactant activity, assessed as its ability to lower the surface tension of a pulsating bubble, and *in vivo* assays utilizing fetal rabbits, have both been described in detail previously by Revak, et al, Am. Rev. Respir. Dis., 134:1258-1265 (1986).

### 8. Morphometric Analyses

Fetal rabbit lungs, inflated to 30 cm H₂O and then deflated to 10 cm H₂O, were submerged in 10% formalin for 72 hours. Paraffin sections were oriented from apex to base and 5 micron sections taken anterior to posterior. After hematoxylin and eosin staining, 10 fields (100 x) were point-counted from apex to base on multiple sections. Standardized morphometric methods (Weiber, in "Stereological Methods," Vol. I, Academic Press, New York, pp. 33-58, 1979) were used to determine ratios of lung interstitium to air spaces for each treatment group. Intersections of alveolar perimeters were also determined.

### 9. Phospholipid phosphorus Assays

Phospholipids were quantitated according to the method of Bartlett, J. Biol. Chem., 234:466-468 (1959).

### 10. Amino Acid Analysis

Triplicate samples for amino acid compositions were hydrolyzed with HCl at 110°C for 24 hours, with HCl at 150°C for 24 hours, or in performic acid at 110°C for 24 hours followed by HCl hydrolysis at 110°C for 24 hours. Analyses were performed on a Beckman model 121-M amino acid analyzer (Beckman Instruments, Fullerton, CA). Tryptophan was not determined.

### 11. Amino Acid Sequencing

Vapor-phase protein sequencing was performed on an Applied Biosystems 470A Amino Acid Sequencer (Applied Biosystems, Inc., Foster City, CA) with an on-line Model 120A HPLC.

### B. Results

The synthetic surfactants illustrated in Table 2 hereinbelow were prepared as indicated in the table. Each of the exemplary synthetic surfactants indicated in Table 2 was assayed for surfactant activity as evidenced by their ability to reduce surface tension *in vitro* using the "bubble assay" of Enhorning as described above.

The results of this study indicate that a subject polypeptide, when admixed with pharmaceutically acceptable phospholipids, forms a synthetic pulmonary surfactant that has greater surfactant activity than the phospholipids alone, as evidenced by the lower ΔP values. Typically 10% to 80% lower ΔP values were obtained using the polypeptides.

Each polypeptide was admixed with the indicated solvent at a concentration of 2.5 mg of polypeptide per ml of solvent. The resulting admixture was observed to determine whether a solution or a suspension of insoluble polypeptide was formed. Those admixtures forming a suspension were further admixed by water bath sonication for 10 seconds to form a very fine suspension, sufficient for pipetting using glass pipettes.

After admixture with solvent, each peptide was admixed with phospholipids (PL), DPPC:PG, 3:1, in chloroform in a glass tube so that the amount of polypeptide added equaled one-tenth (10% by weight) of the amount of PL added, to form a synthetic surfactant according to either method A, B or C.

Each of the synthetic surfactants was then assayed for surfactant activity as evidenced by their ability to reduce surface tension *in vitro* in the bubble assay performed as described above. The pressure gradient (ΔP) is a measure of surfactant activity in the polypeptide-PL third admixture which was determined using an Enhorning Surfactometer as described above. Measurements were obtained at time points of 15 seconds (15"), 1 minute (1') and 5 minutes (5') and are expressed as a mean of three independent measurements of the indicated polypeptide-PL admixture. Pressure gradient measurements for comparable samples of PL alone (PL) and natural human surfactants were determined as controls.

The results of this study are shown in Table 2.

**TABLE 2**

| Peptide | Solvent | (1)Admixture Formed | (2)Phospholipid Admixture Method | (3) of PL mg/ml | (4) Pressure Gradient | | |
|---|---|---|---|---|---|---|---|
| | | | | | 15" | 1' | 5' |
| DL4 (31 mer) | 47% chloroform 53% methanol | solution | C- | 4 | 2.00 | 1.80 | 1.30 |
| | | | | | | | |
| RL4 | 32% chloroform 68% methanol | solution | C- | 4 | 0.58 | 0.65 | 0.33 |
| | | | | | | | |
| RL8 | 19% chloroform 81% methanol | suspension | C+ | 10 | 0.68 | 0.69 | 0.19 |
| | | | | | | | |
| RRL7 | 49% chloroform 51% methanol | solution | C- | 4 | 1.65 | 1.25 | 1.00 |
| | | | | | | | |
| RCL-1 | 79% chloroform 21% methanol | suspension | C+ | 10 | 0.50 | 0.59 | 0.06 |
| | | | | | | | |
| RCL-2 | 67% chloroform 33% methanol | suspension | C+ | 10 | 0.00 | 0.00 | 0.00 |
| | | | | | | | |
| RCL-3 | 75% chloroform 25% methanol | suspension | C+ | 10 | 0.55 | 0.51 | 0.33 |
| | | | | | | | |
| PL | | | C+ | 10 | >2.50 | >2.50 | 2.33 |
| | | | | | | | |
| Natural Human Surfactant | | | | 10 | 1.06 | 0.89 | 0.79 |

| | | | | | | | |
|---|---|---|---|---|---|---|---|
| (1) Whether the initial admixture of peptide and was a solution or a suspension is indicated. | | | | | | | |
| (2) The letters indicate the synthetic surfactant preparation method used. Those methods are described above. A "+" indicates that the final admixture was subjected to a last step of 3 cycles of freezing and thawing. A "-" indicates the step was not performed. | | | | | | | |
| (3) Concentration ("Conc.") of phospholipid (PL) in the final third admixture is indicated in milligrams PL per milliliter admixture (mg/ml). | | | | | | | |
| (4) The pressure gradient is a measure of surfactant activity in the polypeptide-PL final admixture as determined using an Enhorning Surfactometer as described in Example 2. Measurements were obtained at three points of 15 seconds (15"), 1 minute (1') and 5 minutes (5') and are expressed as a mean of 3 independent measurements of the indicated polypeptide-PL admixture. Pressure gradient measurements for comparable samples of PL alone (PL) and natural human surfactant are also shown. (5) These solutions were made at a concentration of 20 mg/ml PL and were diluted to 10 mg/ml prior to testing. | | | | | | | |

These results indicate that a subject polypeptide, when admixed with pharmaceutically acceptable phospholipids, forms a synthetic pulmonary surfactant that has a greater surfactant activity than the phospholipids alone, as demonstrated by the higher volume per given pressure.

### EXAMPLE 2

### In Vivo Assessment of Synthetic Surfactant Activity Using Rabbits

### A. Methods

### 1. Preparation of Synthetic Surfactants

A subject polypeptide was first admixed with solvent as described herein. The resulting admixture was further admixed with phospholipid (PL) so that the amount of polypeptide added was either 3%, 7% or 10% by weight of the amount of PL added as indicated below. The final polypeptide, PL admixture (synthetic surfactant) was formed according to method C using the final freeze thaw step as described in detail in the "Preparation of Synthetic Surfactants" section in Example 1, section 3, except that the final admixture had a concentration of 20 mg phospholipid per ml of final admixture.

### 2. Instillation Protocols

Protocol 1: Fetal rabbits were treated by injection into the trachea of a 0.1 ml solution that contained either a synthetic surfactant prepared as in Example 1, or, either 2 mg of native surfactant prepared as described in Example 1 or 2 mg PL.

Protocol 2: Synthetic surfactant was instilled in rabbit fetal lung by injection into the trachea from a single syringe of the following three components such that the components exit the syringe in the following order: (1) 0.05 ml air; (2) 0.1 ml of a synthetic surfactant prepared as in Example 1, or, either 2 mg of PL or 2 mg of native surfactant; and (3) 0.1 ml air.

Protocol 3: From one syringe, a 0.1 ml aliquot of synthetic surfactant prepared as described in Example 1 (or 2 mg of NS or of PL), was instilled into the rabbit trachea as described above, followed by injection of 0.05 ml lactated Ringer's Solution and 0.2 ml air from a second syringe.

Protocol 4: From one syringe, 0.1 ml of a synthetic surfactant prepared as described in Example 1 (or 2 mg of NS or of PL), 0.15 ml air, 0.1 ml saline, and 0.3 ml air were injected into the trachea as described above. Two subsequent aliquots of 0.3 ml air were given.

Protocol 5: Fetal rabbits were treated by injection into the trachea from a single syringe the following four components such that the four components exit the syringe upon injection in the order listed: (1) 0.2 ml solution that contains either a synthetic surfactant prepared as in Example 1 or either 4 mg of native surfactant, or 4 mg PL; (2) a 0.15 ml volume of air; (3) a 0.1 ml normal saline solution; and (4) a 0.3 ml volume of air. The above injection was then repeated 15 minutes after the first injection.

Protocol 6: Rabbits were treated as described in Protocol 5, except that two subsequent aliquots of 0.3 ml air were given following the initial instillation and no additional instillation was given at 15 min.

### 3. Fetal Rabbit Model for Studying Surfactant Activity

The surfactant activity of exemplary polypeptides of this invention was studied using the methods described in detail previously by Revak, et al, Am. Rev. Respir. Dis., 134:1258-1256 (1986), with the exceptions noted hereinbelow.

Twenty-seven day gestation fetal rabbits were delivered by hysterotomy and immediately injected with 0.05 ml Norcuron (Organon, Inc., NJ) to prevent spontaneous breathing. The fetal rabbits were then weighed and a small cannula was inserted into the trachea by tracheotomy. Synthetic surfactant prepared as described above was then instilled into the fetal rabbit lung by one of the above instillation protocols.

Following instillation the rabbit was placed in a specially designed plethysmograph (containing a Celesco transducer) connected to a ventilator (Baby Bird, Baby Bird Corp., Palm Springs, CA) and the instilled lung was ventilated at a rate of 30 cycles per minute with a peak inspiratory pressure of 25 cm H₂O, a positive end expiratory pressure of 4 cm H₂O and an inspiratory time of 0.5 seconds. In some studies, dynamic compliance measurements were made at various times throughout the ventilation procedure. In others, static compliance measurements were made following ventilation.

Static compliance measurements were made after 30 minutes of ventilation. The animals were removed from the ventilator and the lungs were degassed at -20 cm H₂O in a bell jar under vacuum. Thereafter, the lungs were first inflated and then deflated through a T-connector attached to a tracheostomy tube. The volume of air required to reach static pressures of 5, 10, 15, 20, 25 and 30 cm H₂O was measured during both inflation and deflation phases to generate static pressure to volume curves as a measure of static compliance.

Using the plethysmograph, dynamic compliance measurements were made at various times throughout a 60 minute ventilation period. Computer-assisted data analysis resulted in compliance data expressed as ml of air per cm H₂O per gram of body weight at each time point. Compliance was calculated by the formula below.$\text{Compliance =} \frac{\text{ΔV}}{\text{ΔP}}$
- ΔPₜₚ =: (C)⁻¹•(ΔV) + (R)•(F)
- Pₜₚ =: transpulmonary pressure
- C =: compliance (elastic component - relates change in volume to pressure)
- R =: resistance (relates flow to pressure)
- F =: flow
- V =: volume = the integral of flow with respect to time

The above equation was solved with a multiple linear regression for C and R. The compliance (C) represents the elastic nature of the lungs and the resistance (R) represents the pressure necessary to overcome the resistance to the flow of gas into and out of the lungs.

The studies illustrated in Tables 3-5 were conducted as described above, using a variety of synthetic surfactants prepared according to within-disclosed methods and parameters. Synthetic surfactants consisting of RL2, RL4, RL4-CYS or RL8 peptides, or "multiples" of RL4 (e.g., (RL4)₆R) were compared to a phospholipid surfactant not containing peptide or protein ("control surfactant"). Using the plethysmograph, dynamic compliance measurements were made at various times throughout a 60 minute ventilation period. Data analysis resulted in compliance data expressed as ml of air per cm H₂O per gram of body weight X 10⁶ at each time point. Compliance was calculated by the aforementioned formula.

For the studies illustrated in Tables 3 and 4, the synthetic surfactant solutions were comprised of DPPC, PG and peptide in the following proportions. DPPC: 15 mg/ml; PG: 5 mg/ml; and peptide: 2 mg/ml. In the Table 3 studies, the administered solutions which also contained palmitic acid were designated with a "+" symbol, while those with a "-" did not include palmitic acid. Administration protocol 4 was used in the studies illustrated in Tables 3 and 4.

Table 4 and Figure 4 illustrate the effect of administration of various formulations of RL4-containing surfactant on lung function and demonstrate the results of an effort to determine the optimal length of an RL4-containing peptide. In Table 4, the *in vivo* efficacy of a cysteine-containing synthetic surfactant is also assayed. In Table 4 and Figure 4, the average dynamic compliance value for animals tested using the designated formulation is plotted against time in minutes after surfactant administration. The surfactant was administered as described above, with reference to protocol 4.

### B. Results

Static compliance data using instillation protocols 1 and 5 was gathered and analyzed (data not shown). Improved lung compliance was seen in all lungs treated with natural surfactant or with the synthetic surfactants tested as compared with those lungs treated with phospholipids (PL) alone, with one exception. The synthetic surfactant prepared using p1-15 did not produce improved lung compliance over PL alone when measured by static compliance.

The results of the dynamic compliance studies are illustrated in Tables 3-5 and in Figure 4.

**TABLE 3**

| Dynamic Compliance in ml air/cm H₂O/g body weight x 10⁶ | | | | | | | |
|---|---|---|---|---|---|---|---|
| | Minutes after Surfactant Instillation | | | | | | Sample Given By Protocol # |
| | 10 | 20 | 30 | 40 | 50 | 60 | |
| RL2^{a} | | | | | | | |
| + | 54 | 66 | 120 | 127 | 200 | 247 | 4 |

| RL4 | | | | | | | |
|---|---|---|---|---|---|---|---|
| + | 216 | 140 | 189 | 241 | 289 | 283 | 4 |
| + | 34 | 46 | 88 | 114 | 134 | 193 | 4 |

| RL8 | | | | | | | |
|---|---|---|---|---|---|---|---|
| - | 44 | 80 | 117 | 187 | 226 | 228 | 4 |
| - | 63 | 80 | 102 | 107 | 245 | 154 | 4 |
| + | 27 | 37 | 55 | 96 | 113 | 148 | 4 |

| | | | | | | | |
|---|---|---|---|---|---|---|---|
| ^{a} Solutions referenced with a "+" contain palmitic acid, while those referenced with a "-" do not. | | | | | | | |

**TABLE 4**

| Dynamic Compliance in ml air/cm H₂O/g body weight x 10⁶ | | | | | | | |
|---|---|---|---|---|---|---|---|
| | Minutes after Surfactant Instillation | | | | | | Sample Given By Protocol # |
| | 10 | 20 | 30 | 40 | 50 | 60 | |
| RL2 | | | | | | | |
| | 69 | 147 | 226 | 245 | 239 | 253 | 4 |

| RL8 | | | | | | | |
|---|---|---|---|---|---|---|---|
| | 72 | 442 | 442 | 489 | 464 | 464 | 4 |
| | 79 | 110 | 135 | 126 | 149 | 169 | 4 |

**TABLE 5**

| Dynamic Compliance in ml air/cm H₂O/g body weight x 10⁶ | | | | | | | |
|---|---|---|---|---|---|---|---|
| | Minutes after Surfactant Instillation | | | | | | Sample Given By Protocol # |
| | 10 | 20 | 30 | 40 | 50 | 60 | |
| RL4-CYS | | | | | | | |
| | 20 | 31 | 31 | 47 | 47 | 78 | 4 |
| | 34 | 40 | 34 | 48 | 54 | 71 | 4 |
| | 40 | 40 | 48 | 51 | 66 | n/d^{b} | 4 |

| (RL4)₈R | | | | | | | |
|---|---|---|---|---|---|---|---|
| | 75 | 109 | 173 | PTX^{c} | PTX | PTX | 4 |
| | 32 | 51 | 32 | 37 | 37 | 51 (PTX) | 4 |
| | 42 | 50 | 54 | 65 | 69 | 84 | 4 |
| | 46 | 50 | 50 | 54 | 54 | n/d | 4 |

| (RL4)₇R | | | | | | | |
|---|---|---|---|---|---|---|---|
| | 35 | 39 | 53 | 53 | 74 | 88 | 4 |
| | 30 | 42 | 54 | 83 | 95 | 107 | 4 |
| | 37 | 44 | 55 | 55 | 52 | 55 | 4 |

| (RL4)₆R | | | | | | | |
|---|---|---|---|---|---|---|---|
| | 46 | 39 | 74 | 106 | 124 | 135 | 4 |
| | 80 | 92 | 112 | 141 | 149 | 143 | 4 |
| | 40 | 52 | 60 | 65 | 65 | 60 (PTX) | 4 |

| (RL4)₅R | | | | | | | |
|---|---|---|---|---|---|---|---|
| | 15 | 39 | 66 | 123 | 112 | 73 (PTX) | 4 |
| | 35 | 41 | 56 | 56 | 32 | 53 | 4 |
| | 39 | 85 | 72 | 92 | 82 | 99 | 4 |

| (RL4)₄R | | | | | | | |
|---|---|---|---|---|---|---|---|
| | 38 | 91 | 140 | 206 | 251 | 297 | 4 |
| | 39 | 41 | 56 | 56 | 32 | 53 | 4 |
| | 38 | 44 | 44 | 48 | 52 | 52 | 4 |

| | | | | | | | |
|---|---|---|---|---|---|---|---|
| ^{b} n/d = not determined | | | | | | | |
| ^{c} PTX = pneumothorax | | | | | | | |

As shown in Tables 3-5, each of the synthetic surfactants (and natural surfactant) improved dynamic compliance values in comparison to phospholipid alone.

### C. Discussion

The *in vivo* compliance studies demonstrate that the use of a number of exemplary synthetic surfactants of this inventions resulted in enhanced compliance in comparison to phospholipid alone for each of the assayed synthetic surfactants. Thus, the proteins and polypeptides of this invention when admixed with pharmaceutically acceptable phospholipids form synthetic surfactants that have greater surfactant activity than phospholipid alone. Use of the synthetic surfactants is advantageous in producing improved compliance values *in vivo*.

The studies described herein and depicted in Tables 3-5 and Figure 4 demonstrate that synthetic surfactants display marked therapeutic utility. For example, the dynamic compliance values observed for all the synthetic surfactants significantly exceed those values typically obtained with phospholipid alone. Moreover, as dynamic compliance tests are clearly indicators of *in vivo* efficacy in mammals, including humans, it is not surprising that the herein-described data correlates well with the primate data discussed in Example 3 hereinbelow.

Furthermore, the therapeutic efficacy demonstrated via these *in vivo* dynamic compliance studies is consistent with the results noted when these same compositions were tested using the "pulsating bubble" *in vitro* assays described and illustrated in Example 1 above. The "pulsating bubble" *in vitro* assays of surfactant activity described herein appear to be predictive of *in vivo* efficacy, particularly in light of the fact that the *in vivo* studies described herein demonstrate the therapeutic efficacy of the synthetic surfactants conforming to the formulations disclosed. The significant improvement in dynamic compliance values observed in these *in vivo* studies underscores the therapeutic value of the synthetic surfactants prepared and utilized as disclosed herein.

### EXAMPLE 3

### In Vivo Assessment of Synthetic Surfactant Activity Using Primates

### A. Methods

### 1. Preparation of Synthetic Surfactants

A subject polypeptide was first admixed with solvent as described in Example 2. The resulting admixture was further admixed with phospholipid (PL) so that the amount of polypeptide added was either 3%, 7% or 10% by weight of the amount of PL added as indicated below. The final polypeptide, PL admixture (synthetic surfactant) was formed according to method C using the final freeze thaw step as described in detail in the "Preparation of Synthetic Surfactants" section in Example 1, section 3, except that the final admixture had a concentration of 20 mg phospholipid per ml of final admixture.

### 2. Fetal Monkey Model for Studying Surfactant Activity

*In vivo* studies were initiated to confirm the efficacy of the novel peptides (and synthetic surfactants containing those peptides) described in the above-noted application. Surfactants consisting of phospholipid and RL4 or KL4 peptides were compared to a phospholipid surfactant not containing peptide or protein ("control surfactant").

Following standard protocols, fetal rhesus monkeys of about 128-131 days gestation were delivered. An endotracheal tube was then inserted through a tracheostomy. Each monkey was subsequently connected to a ventilator and a catheter was placed in the umbilical artery for the purpose of measuring blood gases and blood pressure; a second catheter was placed in the umbilical vein for the purpose of infusing the monkey with a nutrient/hydrating solution (D10W). After the animals were stabilized, X-rays were taken to assess the presence and extent of respiratory distress syndrome (RDS). Various parameters were adjusted to maintain the oxygen pressure (pO₂) in the range of 50-70 torr and the carbon dioxide pressure (pCO₂) at 45-50 torr. Pulse oximetry was used to continuously monitor hemoglobin saturation of arterial blood.

As an index of oxygenation, a/A (arterial/alveolar) O₂ ratios were calculated at the time of each measurement of arterial pO₂. These values, along with radiographic evidence and clinical assessments of the monkeys' condition, allowed determination of the presence and severity of RDS. An a/A ratio of 0.2 to 0.4 confirms the presence of RDS; values below 0.2 are indicative of severe RDS.

Once the diagnosis of RDS was established, each monkey was maintained with ventilatory support, generally for 2 hours. Peptide-containing or control surfactants were then administered via a feeding tube inserted down the endotracheal tube. One-half the dose of synthetic surfactant was given with the animal held on its right side, and the other half while the animal was held on its left side. In the experiments illustrated in Figures 2A and 2B, blind trials were conducted; i.e., the individual instilling the synthetic surfactant was not informed as to which surfactant (i.e., peptide-containing surfactant or phospholipid control surfactant) the animal was receiving.

Ventilatory support was maintained for another 6-12 hours and each animal's condition was continuously monitored. Mid-experiment and pre-terminal X-rays were taken as well. When the experiment terminated, each animal was sacrificed (by phenobarbital injection) and a necropsy performed.

### B. Results

The data generated from the above-described studies are illustrated in Figures 1, 2 and 3. The Figures illustrate the following.

Figure 1 illustrates the effect of administration of RL4-containing surfactant on lung function. In Fig. 1A, the index of oxygenation (a/A) is plotted against time subsequent to delivery of the animal, in hours. The surfactant was administered in split dosage, as described above, about 28 hours after delivery. In Figure 1B, for a second monkey, RL4-containing synthetic surfactant was administered in split dosage, as described above, during the first 2.5 hours after delivery. As in the first monkey (Fig. 1A), the a/A ratio dramatically improved in the hours following peptide-containing surfactant administration.

In Figures 2A and B, the effect of KL4-containing synthetic surfactant administration on lung function is shown. In Fig. 2A and 2B, the data for eight monkeys are shown. Those which were later confirmed to have received KL4-containing synthetic surfactant were identified as Monkey Nos. 6, 7, 8, and 10, while those monkeys receiving another surfactant (i.e., one not containing a surfactant polypeptide of the present invention) were Monkey Nos. 3, 5, 9, and 11. In all plots, a/A is plotted against hours after birth, with the time of administration of surfactant indicated.

Values for final oxygen revealed that the animals receiving peptide-containing surfactant tolerated low concentrations of inspired oxygen; their pCO₂ levels were low, final pH of their blood was normal, and their lungs were expanded as determined by gross and microscopic inspection (the studies were blinded as noted above). In each case, X-rays performed immediately before surfactant administration demonstrated clouding of the lung fields, but only in the four monkeys receiving KL4-containing surfactant did the lung fields clear by 8-10 hours after birth.

In Monkey No. 8, the lack of improvement in a/A ratio was found to be the result of an interventricular septal defect allowing right-to-left shunting of oxygen-poor venous blood. In all other monkeys receiving the presently-disclosed synthetic surfactants, recovery from RDS was dramatic.

Each primate's final FiO₂, final pCO₂, final pH, and lung expansion (gross and microscopic) was measured as well. The data collected and recorded are as follows.

Monkey No. 3 (Fig. 2A-1) : Final FiO₂ - 50; final pCO₂ - 79.4; final pH - 7.21; lung expansion, gross - 0; lung expansion, microscopic - 0.

Monkey No. 5 (Fig. 2A-2): Final FiO₂ - 90; final pCO₂ - 58.6; final pH- 7.17; lung expansion, gross - 0; lung expansion, microscopic - 0.

Monkey No. 6 (Fig. 2A-3): Final FiO₂ - 21; final pCO₂ - 39.4; final pH- 7.39; lung expansion, gross - 4+; lung expansion, microscopic - 4+.

Monkey No. 7 (Fig. 2A-4): Final FiO₂ - 21; final pCO₂ - 29.3; final pH- 7.47; lung expansion, gross - 4+; lung expansion, microscopic - 3+.

Monkey No. 8 (Fig. 2B-1): Final FiO₂ - 21; final pCO₂ - 32.1; final pH- 7.42; lung expansion, gross - 4+; lung expansion, microscopic - 4+.

Monkey No. 9 (Fig. 2B-2): Final FiO₂ - 100; final pCO₂ - 150.1; final pH - 6.90; lung expansion, gross - 0; lung expansion, microscopic - 0.

Monkey No. 10 (Fig. 2B-3): Final FiO₂ - 21; final pCO₂ - 31.5; final pH- 7.42; lung expansion, gross - 2+; lung expansion, microscopic - 3+.

Monkey No. 11 (Fig. 2B-4) : Final FiO₂ - 50; final pCO₂ - 55.6; final pH- 7.31; lung expansion, gross - 0; lung expansion, microscopic - 0.

Figure 3 illustrates the gradual withdrawal of oxygen over time (in hours) in Monkey No. 13, subsequent to administration of KL4-containing surfactant. As noted in Fig. 3, KL4-containing surfactant was administered between about one and two hours after delivery. At time zero and 100% inspired oxygen (FiO₂ = 1.0), the animal was receiving 100% oxygen; at 22-25 hours, the animal was receiving 20% oxygen -- that is, room air.

### C. Discussion

From these data it was concluded that the synthetic surfactants of the present invention are not merely therapeutically useful, they produce a dramatic improvement in the recipient's lung function within a relatively brief period of time. This utility is well-illustrated by the fact that animals receiving a synthetic surfactant containing a peptide conforming to the formulations disclosed in the above-referenced application fully recovered from RDS after administration of RL4 or KL4 surfactant.

In addition, the data indicate that administration of these novel peptide-containing surfactants allows recovery sufficient to warrant the removal of enriched oxygen administration once the a/A ratio indicates that the organism is no longer experiencing respiratory distress.

The experimental results discussed above are consistent with the information ascertained from the "pulsating bubble" assays, which provide a valuable *in vitro* model of *in vivo* efficacy (see Example 1), and the *in vivo* dynamic compliance studies (see Example 2). In addition, the "bubble" assay results appeared to predict that the synthetic surfactants (including, for example, RL4 and KL4) would demonstrate therapeutic efficacy, as illustrated herein.

The foregoing specification, including the specific embodiments and examples, is intended to be illustrative of the present invention and is not to be taken as limiting. Numerous other variations and modifications can be effected without departing from the true spirit and scope of the present invention.

## Claims

1. A polypeptide having an amino acid residue sequence represented by the formula: or respectively SEQ ID NOs 7-9.

2. A polypeptide having an amino acid residue sequence represented by the formula:

3. A synthetic pulmonary surfactant comprising a pharmaceutically acceptable phospholipid admixed with a polypeptide according to claim 1 or claim 2.

4. A synthetic pulmonary surfactant comprising two pharmaceutically acceptable phospholipids admixed with a fatty acid and a polypeptide according to claim 1 or claim 2.

5. A method of making a therapeutic medicament useful for treating respiratory distress syndrome, comprising mixing one or more pharmaceutically acceptable phospholipids with a therapeutically effective amount of a polypeptide according to claim 1 or claim 2.

## Patentansprüche

1. Polypeptid mit einer Aminosäuresequenz, die durch die folgenden Formeln dargestellt ist: oder bzw. durch die SEQ ID Nrn. 7-9.

2. Polypeptid mit einer Aminosäuresequenz, die durch die folgende Formel dargestellt ist:

3. Synthetisches, pulmonales, oberflächenaktives Mittel, welches ein pharmazeutisch akzeptables Phospholipid in Beimischung zu einem Polypeptid nach Anspruch 1 oder Anspruch 2 umfasst.

4. Synthetisches, pulmonales, oberflächenaktives Mittel, welches zwei pharmazeutisch akzeptable Phospholipide in Beimischung mit einer Fettsäure und einem Polypeptid nach Anspruch 1 oder Anspruch 2 umfasst.

5. Verfahren zur Herstellung eines therapeutischen, zur Behandlung des Atemnotsyndroms nützlichen Medikamentes, welches das Mischen von einem oder mehreren pharmazeutisch akzeptablen Phospholipiden mit einer therapeutisch wirksamen Menge eines Polypeptides nach Anspruch 1 oder Anspruch 2 umfasst.

## Revendications

1. Polypeptide ayant une séquence de résidus d'acides aminés représentée par la formule: ou respectivement SEQ ID Nos 7-9.

2. Polypeptide ayant une séquence de résidu d'acides aminés représentée par la formule:

3. Agent tensioactif pulmonaire synthétique comprenant un phospholipide pharmaceutiquement acceptable en mélange avec un polypeptide selon la revendication 1 ou la revendication 2.

4. Agent tensioactif pulmonaire synthétique comprenant deux phospholipides pharmaceutiquement acceptables en mélange avec un acide gras et un polypeptide selon la revendication 1 ou la revendication 2.

5. Méthode de production d'un médicament thérapeutique utile pour le traitement du syndrome de détresse respiratoire, consistant à mélanger un ou plusieurs phospholipides pharmaceutiquement acceptables avec une quantité thérapeutiquement efficace d'un polypeptide selon la revendication 1 ou la revendication 2.


### SEQUENCE LISTING

### (1) GENERAL INFORMATION:

(i) APPLICANT:
   Cochrane, Charles G
   Revak, Susan D
(ii) TITLE OF INVENTION: SYNTHETIC PULMONARY SURFACTANT PEPTIDES
(iii) NUMBER OF SEQUENCES: 10
(iv) CORRESPONDENCE ADDRESS:
   (A) ADDRESSEE: The Scripps Research Institute, Office of Patent Counsel
   (B) STREET: 10666 North Torrey Pines Road, Mail Drop TPC8
   (C) CITY: La Jolla
   (D) STATE: California
   (E) COUNTRY: US
   (F) ZIP: 92037
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: Patentin Release #1.0, Version #1.25
(vi) CURRENT APPLICATION DATA:
   (A) APPLICATION NUMBER: PCT/US92/04537
   (B) FILING DATE: 01-JUN-1992
   (C) CLASSIFICATION:
(vii) PRIOR APPLICATION DATA:
   (A) APPLICATION NUMBER: US 07/715,397
   (B) FILING DATE: 14-JUN-1991
(viii) ATTORNEY/AGENT INFORMATION:
   (A) NAME: Bingham, Douglas A
   (B) REGISTRATION NUMBER: 32,457
   (C) REFERENCE/DOCKET NUMBER: SCR1025P
(ix) TELECOMMUNICATION INFORMATION:
   (A) TELEPHONE: 619-554-2937
   (B) TELEFAX: 619-554-6312

### (2) INFORMATION FOR SEQ ID NO:1:

(i) SEQUENCE CHARACTERISTICS:
   (A) LENGTH: 21 amino acids
   (B) TYPE: amino acid
   (D) TOPOLOGY: linear
(ii) MOLECULE TYPE: peptide
(xi) SEQUENCE DESCRIPTION: SEQ ID NO:1:
   Arg Leu Leu Leu Leu Arg Leu Leu Leu Leu Arg Leu Leu Leu Leu Arg
   1               5                   10                  15
   Leu Leu Leu Leu Arg
               20

### (2) INFORMATION FOR SEQ ID NO:2:

(i) SEQUENCE CHARACTERISTICS:
   (A) LENGTH: 21 amino acids
   (B) TYPE: amino acid
   (D) TOPOLOGY: linear
(ii) MOLECULE TYPE: peptide
(xi) SEQUENCE DESCRIPTION: SEQ ID NO:2:
   Arg Leu Leu Leu Leu Leu Leu Leu Leu Arg Leu Leu Leu Leu Leu Leu
   1               5                   10                  15
   Leu Leu Arg Leu Leu
               20

### (2) INFORMATION FOR SEQ ID NO:3:

(i) SEQUENCE CHARACTERISTICS:
   (A) LENGTH: 21 amino acids
   (B) TYPE: amino acid
   (D) TOPOLOGY: linear
(ii) MOLECULE TYPE: peptide
(xi) SEQUENCE DESCRIPTION: SEQ ID NO:3:
   Arg Arg Leu Leu Leu Leu Leu Leu Leu Arg Arg Leu Leu Leu Leu Leu
   1 5 10 15
   Leu Leu Arg Arg Leu
   20

### (2) INFORMATION FOR SEQ ID NO:4:

(i) SEQUENCE CHARACTERISTICS:
   (A) LENGTH: 19 amino acids
   (B) TYPE: amino acid
   (D) TOPOLOGY: linear
(ii) MOLECULE TYPE: peptide
(xi) SEQUENCE DESCRIPTION: SEQ ID NO:4:
   Arg Leu Leu Leu Leu Cys Leu Leu Leu Arg Leu Leu Leu Leu Cys Leu
   1 5 10 15
   Leu Leu Arg

### (2) INFORMATION FOR SEQ ID NO:5:

(i) SEQUENCE CHARACTERISTICS:
   (A) LENGTH: 21 amino acids
   (B) TYPE: amino acid
   (D) TOPOLOGY: linear
(ii) MOLECULE TYPE: peptide
(xi) SEQUENCE DESCRIPTION: SEQ ID NO:5:
   Arg Leu Leu Leu Leu Cys Leu Leu Leu Arg Leu Leu Leu Leu Cys Leu
   1 5 10 15
   Leu Leu Arg Leu Leu
   20

### (2) INFORMATION FOR SEQ ID NO:6:

(i) SEQUENCE CHARACTERISTICS:
   (A) LENGTH: 28 amino acids
   (B) TYPE: amino acid
   (D) TOPOLOGY: linear
(ii) MOLECULE TYPE: peptide
(xi) SEQUENCE DESCRIPTION: SEQ ID NO:6:
   Arg Leu Leu Leu Leu Cys Leu Leu Leu Arg Leu Leu Leu Leu Cys Leu
   1               5                   10                  15
   Leu Leu Arg Leu Leu Leu Leu Cys Leu Leu Leu Arg
               20                  25

### (2) INFORMATION FOR SEQ ID NO:7:

(i) SEQUENCE CHARACTERISTICS:
   (A) LENGTH: 21 amino acids
   (B) TYPE: amino acid
   (D) TOPOLOGY: linear
(ii) MOLECULE TYPE: peptide
(xi) SEQUENCE DESCRIPTION: SEQ ID NO:7:
   Lys Leu Leu Leu Leu Lys Leu Leu Leu Leu Lys Leu Leu Leu Leu Lys
   1               5                   10                  15
   Leu Leu Leu Leu Lys
                   20

### (2) INFORMATION FOR SEQ ID NO:8:

(i) SEQUENCE CHARACTERISTICS:
   (A) LENGTH: 21 amino acids
   (B) TYPE: amino acid
   (D) TOPOLOGY: linear
(ii) MOLECULE TYPE: peptide
(xi) SEQUENCE DESCRIPTION: SEQ ID NO:8:
   Lys Leu Leu Leu Leu Leu Leu Leu Leu Lys Leu Leu Leu Leu Leu Leu
   1 5 10 15
   Leu Leu Lys Leu Leu
   20

### (2) INFORMATION FOR SEQ ID NO:9:

(i) SEQUENCE CHARACTERISTICS:
   (A) LENGTH: 21 amino acids
   (B) TYPE: amino acid
   (D) TOPOLOGY: linear
(ii) MOLECULE TYPE: peptide
(xi) SEQUENCE DESCRIPTION: SEQ ID NO:9:
   Lys Lys Leu Leu Leu Leu Leu Leu Leu Lys Lys Leu Leu Leu Leu Leu
   1 5 10 15
   Leu Leu Lys Lys Leu
   20

### (2) INFORMATION FOR SEQ ID NO:10:

(i) SEQUENCE CHARACTERISTICS:
   (A) LENGTH: 21 amino acids
   (B) TYPE: amino acid
   (D) TOPOLOGY: linear
(ii) MOLECULE TYPE: peptide
(xi) SEQUENCE DESCRIPTION: SEQ ID NO:10:
   Asp Leu Leu Leu Leu Asp Leu Leu Leu Leu Asp Leu Leu Leu Leu Asp
   1 5 10 15
   Leu Leu Leu Leu Asp
   20